# FASCICULE DE BREVET EUROPEEN

(11) **EP 0 573 618 B1**
(45) Date de publication et mention de la délivrance du brevet: **08.02.1995**
(21) Numéro de dépôt: 92918270.7
(22) Date de dépôt: 01.09.1992
(51) Int. Cl.: A61B 17/39, A61B 17/41

(54) **DISPOSITIF D'EPILATION ELECTRONIQUE A HAUTE FREQUENCE**
ELEKTRONISCHE HOCHFREQUENZVORRICHTUNG ZUR HAARENTFERNUNG
HIGH FREQUENCY ELECTRONIC DEPILATORY DEVICE

(30) Priorité: 05.09.1991 FR 9111002
(43) Date de publication de la demande: 15.12.1993
(73) Titulaire: Bernaz, Gabriel, CH-1227 Carouge (CH)
(72) Inventeur: Bernaz, Gabriel, CH-1227 Carouge (CH)
(74) Mandataire: Munzinger, John Patrick
(86) Numéro de dépôt international: EP9202010
(87) Numéro de publication internationale: WO9304636

(56) Documents cités:
- EP-A- 0 384 640
- WO-A-80/02640
- CH-A- 109 802
- DE-A- 1 717 117
- FR-A- 2 539 622

## Description

L'invention se rapporte au traitement de la peau par des moyens électriques à haute fréquence, en particulier pour l'épilation dite "définitive" ou "longue durée", ainsi que pour la repousse des cheveux.

Il existe trois procédés d'épilation électrique haute fréquence.

L'un de ces procédés consiste à dévitaliser la racine du poil en introduisant dans le follicule pileux une aiguille jusqu'à proximité du bulbe afin d'appliquer directement sur celui-ci une énergie haute fréquence. Ce procédé est délicat et demande une main d'oeuvre spécialisée. L'utilisation d'une aiguille creuse par laquelle, après l'application d'un courant haute-fréquence, passe un liquide qui s'attaque à la racine est décrite par DE-A-1 717 117. Cet appareil n'est adapté que pour de grosses pilosités.

Dans le second procédé, le poil est pris à sa base avec une pincette reliée à un générateur haute fréquence. L'extraction du poil est progressive après plusieurs dizaines de secondes. Par exemple, WO80/02640 décrit l'utilisation d'une solution conductrice qui mouille le poil dans le follicule et l'application d'un courant HF au moyen d'une pincette. L'application du courant dure jusqu'à 20 secondes. Ce procédé est peu efficace.

Dans le troisième procédé, du courant à haute fréquence est appliqué au moyen d'un disque plat sur la peau. Ce procédé s'est également avéré inefficace car il ne permet pas une action ponctuelle. Par ailleurs, l'utilisation d'une solution oxydante et l'application d'une basse tension de 4-5 volts au moyen d'électrodes plates, selon CH-109802, est aussi un procédé inefficace.

En ce qui concerne la repousse des cheveux, on a déjà proposé l'utilisation de sondes isolées à hautes fréquences dans le but de faire pénétrer un produit favorisant la repousse des cheveux. Les résultats se sont avérés peu concluants.

FR-A-2 539 622 décrit une composition pour le traitement du cuir chevelu par électrophorèse constituée par une solution de photosensibilisateur aminovinylique en général dissout dans une solution alcoolique saline, éventuellement avec l'adjonction d'un ou de plusieurs agents ou matières nutritifs. L'électrophorèse a lieu par l'application d'une onde de basse fréquence à deux phases, l'une de fréquence de l'ordre de 50 à 300 Hz sur une onde carrée d'une fréquence de l'ordre de 0.5 à 2 Hz.

D'autre part, EP-A-0 384 804 décrit un masque conducteur comprenant une composition apte à faire prise, composé d'un gel stable à l'abri de l'air mais polymérisant au contact de l'air. Lorsque ce produit est appliqué sur la peau, il prend consistance et pendant un certain temps, on peut passer une tension pulsée à une basse fréquence comprise entre 40 et 110 Hz à but thérapeutique ou esthétique.

Ces traitements à basse fréquence ne font pas partie de l'objet de l'invention qui concerne un procédé de traitement cosmétique par l'application sur la peau d'un produit traitant destiné à agir sur les racines des poils ou des cheveux, et l'application d'un courant électromagnétique de haute fréquence.

L'invention est basée sur la constatation qu'en mélangeant un gel conducteur de type usuel pour l'application de sondes à ultrasons contre la peau avec un produit traitant, par exemple une lotion à effet d'atrophie sur les racines des poils, il est possible par induction transcutanée à haute fréquence de faire pénétrer le produit traitant dans les pores et ainsi d'effectuer un traitement.

On aboutit ainsi à un nouveau procédé de traitement, notamment cosmétique, de la peau, par exemple en vue d'une épilation longue durée, et qui de surcroit permet une application ponctuelle et efficace au niveau des follicules sans intervention manuelle délicate. Bien entendu, la même technique peut également convenir pour introduire dans la peau d'autres produits cosmétiques, par exemple pour la repousse des cheveux ou contre les rides, ainsi que des produits pharmaceutiques, par exemple pour les soins d'acné, séborrhée, plaies ou douleurs.

L'invention, telle que définie dans les revendications, a pour objet un procédé et un appareil pour l'épilation longue durée et autres traitements de la peau, qui sont d'un emploi facile et efficace. D'autre part, l'invention a pour objet l'utilisation de l'appareil pour l'épilation ou la repousse des cheveux, l'utilisation cosmétique du mélange gel/produit, ainsi que le mélange gel et produit utilisé avec l'appareil ou lors du procédé, et un nouveau circuit électrique générateur de haute fréquence.

L'appareil selon l'invention comprend un organe maniable de contact avec la peau ayant un corps non-conducteur pourvu d'une surface d'appui destinée à être appliquée sur la peau. Cette surface comporte une pluralité de points conducteurs d'émission électromagnétique distincts formés par exemple des parties exposées des spires d'un solénoïde noyé dans le corps de l'organe de contact. Ces points débouchent dans des orifices dans cette surface, de préférence en retrait par rapport à celle-ci, de manière que, en cours d'utilisation de l'appareil, ils puissent contacter du gel conducteur chargé appliqué sur la peau. Ces points d'émission électromagnétique distincts émettent du courant à haute fréquence, avantageusement un courant émetteur pur fourni par un circuit électrique oscillant haute fréquence de puissance.

L'organe maniable forme donc une sonde focalisée dont l'energie haute fréquence assure une action ponctuelle à travers le gel chargé. En disposant les points d'émission distincts d'une manière adéquate sur la surface d'appui, on obtient une action simultanée sur tous les follicules d'une zone étendue da la peau. Par exemple, les points d'émission distincts seront alignés en une ou plusieurs rangées le long d'une surface d'appui oblongue de dimensions adaptées à la partie du corps à traiter. De préference, il est prévu plusieurs organes de contact interchangeables, ayant des surfaces d'appui de forme et/ou de grandeur différentes, et éventuellement aussi un embout à aiguille interchangeable, permettant ainsi des traitements propres à toutes sortes de pilosités.

Le circuit électrique génerateur de haute fréquence comprend avantageusement un circuit oscillant haute fréquence de puissance comportant un transistor monté en oscillateur de puissance en combinaison avec des bobines de self inductance double spirale en carré, et une électrode permettant d'ajouter l'impédance du corps d'une personne traitée à celle des bobines de self inductance de manière à élever la fréquence du courant émetteur lors de l'utilisation.

D'autres caractéristiques de l'invention ressortiront à la lecture de la description qui suit, donnée à titre d'exemple, en se référant aux dessins dans lesquels:
la Figure 1 est un schéma illustrant un traitement d'épilation selon l'invention,
la Figure 2 est une vue schématique en coupe axiale d'un organe de contact d'un appareil selon l'invention, dont la Figure 2a montre un détail à plus grande échelle,
la Figure 3 est une vue de face de l'organe de contact de la Figure 2, dont la Figure 3a montre un détail à plus grande échelle, et
la Figure 4 est un diagramme du circuit électrique de l'appareil selon l'invention.

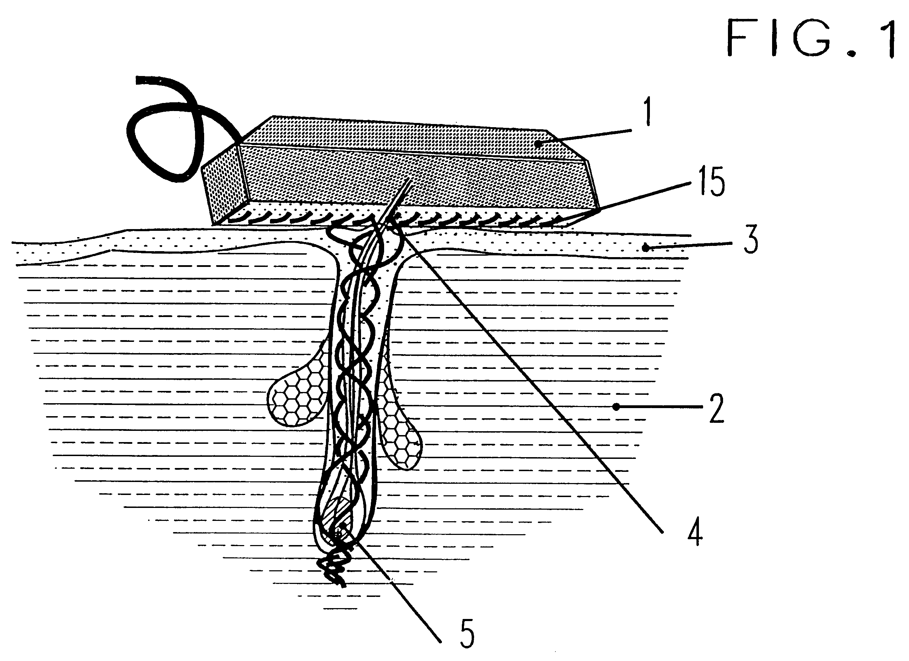

La Figure 1 représente, de façon schématique, une sonde 1 appliquée sur la peau 2 recouverte d'un gel conducteur et mouillant 3 comportant un produit bio-actif. Par l'application d'une énergie à haute fréquence émise par les points d'émission 15 de la sonde 1, une solution conductrice provenant du gel chargé 3 et comportant le produit bio-actif pénètre dans le pore d'un poil 4 jusqu'au bulbe 5.

Le gel chargé 3 est composé d'un gel conducteur d'un type usuel pour le couplage ultrasonique d'électrodes sur la peau, en mélange avec un produit bio-actif. Le gel a un pH neutre et est, par exemple, à base d'un polymère de l'ester 5-bromo-5-nitro-1,3-dioxane-2-carboxyvinylique. La composition du produit bio-actif dépend de l'action souhaitée. Pour une action dépilatoire, on peut choisir un produit à effet d'atrophie progressif de la racine du poil, par exemple une lotion post-épilatoire du type utilisé d'habitude immédiatement après, ainsi que les jours suivants, une épilation à la cire. De telles lotions comportent des extraits de plantes, des huiles essentielles, de l'eau déminéralisée et éventuellement d'autres composants, par exemple des polyoxyéthylènes. Ces produits, connus parfois sous la dénomination "modérateur de la repousse des poils", sont sans danger d'utilisation toxique ou autres.

Pour un traitement de la calvitie, on peut mélanger le gel avec, par exemple, du minoxydil, ou un autre produit favorisant la repousse des cheveux. Un mélange de 50:50 vol% de gel et de minoxydil a donné des résultats satisfaisants.

Afin de ne pas compromettre les propriétés conductrices du gel, la part du produit actif ou traitant n'excèdera en général pas 50%, usuellement moins de 25%, en poids du gel (% en poids = % en volume). On peut ajouter de l'alcool, du chlorure de sodium et/ou autres substances pour améliorer la conductivité du produit, et/ou comme agents conservateurs.

Des tests ont démontré que l'application d'une énergie à haute fréquence sur le produit bio-actif seul ou sur le gel seul n'aboutit à aucun effet spécial, tandis qu'en mélange, on obtient une bonne pénétration du produit bio-actif entrainé par la solution conductrice provenant du gel. Il semble que la conduction de l'énergie électromagnétique et électrique suit le chemin de moindre resistance offert par le mélange du gel et de la lotion conductrice appliqué à la surface de la peau, et ensuite autour de la racine du poil ou du cheveu seulement par la lotion conductrice qui pénètre dans le pore. Le gel permet un dégagement progressif de la lotion active et sa pénétration, sous l'action conjuguée des champs électromagnétique et électrique. Cette action synergétique était imprévisible.

Le courant haute fréquence a un excellent coefficient d'absorption dans la peau, car le système se comporte comme un émetteur-récepteur de radio. A cet effet, on emploiera de préférence un courant haute fréquence émetteur pur, exempt de toute harmonique.

L'énergie du courant haute fréquence est focalisée sur la peau par les points d'émission 15 situés sur la partie de la sonde 1 qui est en contact avec le gel chargé 3. Cette énergie suit la solution conductrice et entraîne le produit actif dans le follicule, provoquant un échauffement progressif accompagné d'une dilatation du follicule pileux, ce qui favorise la pénétration du produit actif et augmente l'efficacité de l'action de ce dernier sur la racine du poil. Ceci produit un effet d'ionithermolyse intra-racine du poil, par pénétration effective de la solution conductrice et bio-active.

Il suffit donc de déplacer lentement la sonde 1 pour produire après quelques minutes un assouplissement du follicule pileux et, en cas de traitement dépilatoire, une atrophie de la racine du poil, favorisant ainsi son extraction par le moyen classique de la cire froide, par exemple. Plusieurs séances sont nécessaires pour produire une atrophie définitive du poil.

Pour la repousse des cheveux, le même procédé d'application sert à faire pénétrer le produit traitant dans la racine du cheveu et augmente son efficacité. Ce procédé s'applique non seulement à la repousse des cheveux naturels du cuir chevelu mais également aux cheveux transplantés pour augmenter l'efficacité du transplant.

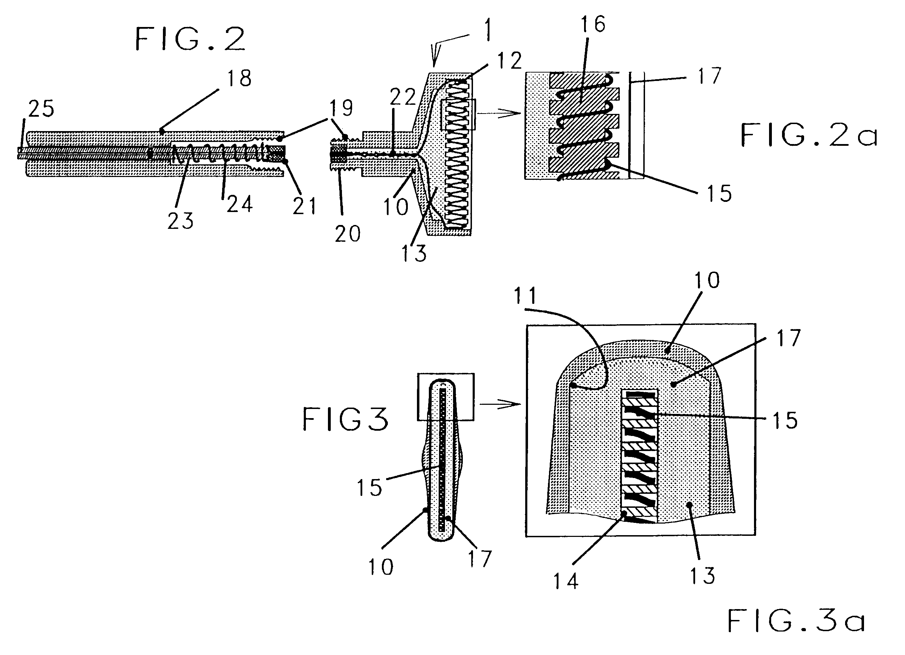

La sonde 1, qui constitue un organe maniable de contact avec la peau 2, est illustrée plus en détail dans les Figures 2 et 3. Elle comporte un corps creux 10 en matière plastique, présentant à l'avant une bouche oblongue 11 et renfermant un solénoïde 12 noyé dans une masse 13 de résine sauf en regard de la bouche 11 où la masse 13 présente une ouverture 14 où débouche une partie 15 de chaque spire du solénoïde 12. Ces parties 15 constituent des points conducteurs d'emission focalisée entrant en contact avec le gel chargé 3.

Le solénoïde 12 est par exemple réalisé en fil de cobalt-nickel amagnétique ayant une section de 0.35 mm², enroulé dans la gorge d'une tige filetée 16 en matière plastique, par exemple de 7 mm diamètre. La gorge a par exemple un pas de 1 mm et son fond est situé à environ 3 mm en retrait de la surface exposée 17 de la masse 13 afin que les points 15 n'entrent pas en contact électrique direct avec la peau lorsque cette surface est appliquée sur elle.

Les points 15 accessibles par l'ouverture 14 forment donc des fenêtres de conduction électrique et électromagnétique vers le gel chargé 3. Ces points 15 sont de préférence alignés en une ou plusieurs rangées le long de la surface oblongue d'appui 17. D'autres dispositions sont possibles, mais la forme allongée avec une seule rangée de points, telle qu'illustrée aux Figures 3 et 3a, a l'avantage d'éviter des interférences éventuelles.

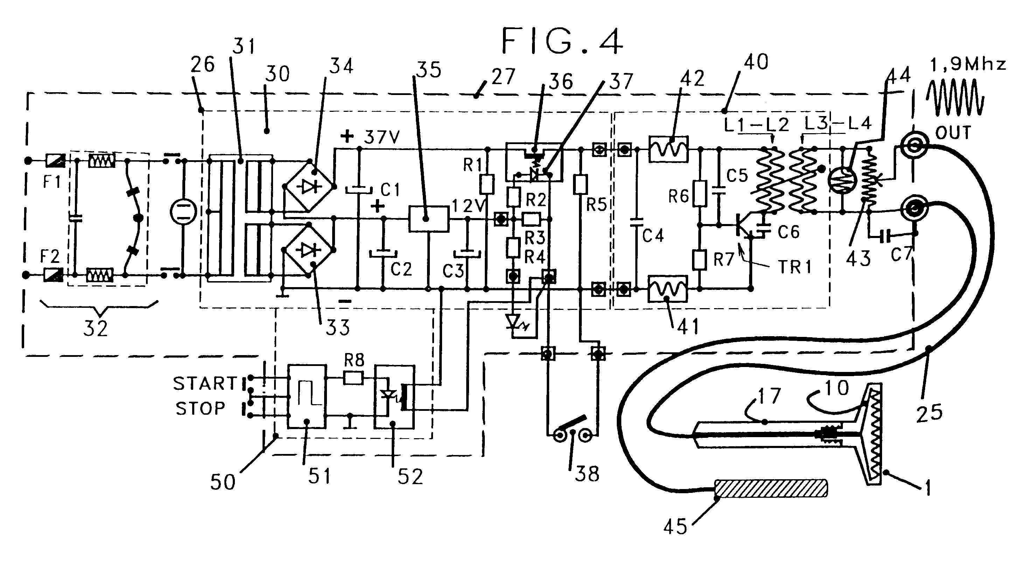

La sonde 1 est fixée de manière amovible sur une poignée 18 au moyen de filetages 19. Ces filetages 19 amènent deux douilles 20 et 21 à entrer en contact, la douille 20, disposée dans le corps 10, étant reliée par des fils 22 aux extrémités du solenoïde 12, alors que la douille 21, disposée de façon coulissante au bout d'une tige 23 à l'intérieur de la poignée 18, est reliée d'abord par un ressort 24 entourant la tige 23 puis par un fil 25 au circuit 27 représenté à la Figure 4.

Pour permettre une action efficace sur les différentes parties du corps et sur les différentes sortes de pilosité, plusieurs sondes 1 de forme et/ou de dimensions différentes peuvent être fixées à choix et de manière amovible sur la poignée 18. Par exemple, on peut prévoir une première sonde longue de 15 mm pour la moustache, une autre longue de 25 mm pour le visage et l'avant du corps, et une troisième longue de 45 mm pour le dos et les jambes. Pour les traitements de grandes surfaces, on peut également prévoir une sonde rectangulaire pourvue de plusieurs rangées de points d'émission bien espacés les uns des autres, avantageusement avec balayage sectoriel du courant électromagnétique et électrique. Il est également possible de prévoir un embout à aiguille, interchangeable avec les sondes 1, permettant l'utilisation dans certains cas du procédé classique d'épilation haute fréquence à aiguille.

Ce circuit 27 est contenu dans un boîtier et comprend un circuit d'alimentation 26 comportant une double alimentation stabilisée 30 suivie d'un circuit oscillateur 40 délivrant en sortie un courant de faible amplitude à très haute fréquence.

De manière conventionnelle, on reconnaît un transformateur d'entrée 31 relié au secteur au travers d'un circuit anti-parasite 32. Un premier enroulement secondaire du transformateur 31 est relié à un pont de diodes 33 créant une tension continue de 12 V stabilisée par des condensateurs C2, C3 et un premier circuit intégré 35. Un second enroulement du transformateur 31 est relié à un second pont de diodes 34 ajoutant une seconde tension d'ordre de 25 V aux 12 V mentionnés précédemment pour créer une tension totale continue de 37 V. Cette seconde tension est regulée par le condensateur C1 et par un second circuit integré 36, lequel est activé ou pas par un circuit 50 de mise en marche et de temporisation, au travers d'une diode émettrice 37.

Ce circuit 50 comprend un circuit integré 51 de création d'intervalles de temps relié à la diode émettrice 37 par une liaison optoélectronique 52.

Le circuit oscillateur 40 comprend un transistor TR1, monté en émetteur commun avec une self L1 - L2, réalisée en double bobinage carré, branchée entre le collecteur du transistor TR1 et la borne positive de l'alimentation, la base du transistor TR1 étant reliée, d'une part, au milieu d'un pont de résistances R6, R7 fixant la tension et, d'autre part, à la borne positive de l'alimentation par un condensateur C5 de 1,2 nF. Un second condensateur C6 de 330 picofarad relie par ailleurs le collecteur et l'émetteur du transistor TR1.

De par l'interaction du condensateur C5 sur la base et de l'impédance de la self L1 - L2 sur le collecteur, le transistor TR1 travaille en oscillateur. Des inductances 41 et 42 empêchent les oscillations du transistor TR1 de remonter dans le circuit d'alimentation 30.

Les oscillations génerées dans l'inductance de la self L1 - L2 sont retransmises avec doublement de la fréquence dans la self secondaire L3 - L4 (selon le même principe qu'un transformateur) dont le circuit est fermé par un potentiomètre 43, avec un limiteur de tension à 50 V ou "shunt" 44 branché en parallèle, permettant de ne prélever qu'une portion de la tension mais toujours à la même fréquence amplifiée. La sortie de ce potentiomètre 43 est reliée à une electrode de base 45 et à la sonde haute fréquence 1. On peut remplacer le potentiomètre 43 par un circuit intégré et un potentiomètre (régulateur de tension) inséré dans le circuit d'alimentation 30.

Le courant haute fréquence est donc produit par le transistor TR1 monté en oscillateur auto-oscillant de puissance.

La bobine de self inductance (self) L1 - L2 est réalisée sur un circuit imprimé à double spirale parallèle en carré, superposée à 1.6 mm. Cette self est placée à une distance de 4 à 6 mm de la deuxième self L3 - L4, aussi en double spirale parallèle en carré, superposée à 1.6 mm. La self L3 - L4 est donc accordée sur la self L1 - L2.

Cette conception de la self permet de faire osciller le transistor TR1 en doubleur de fréquence et de produire une réaction dynamique sur l'oscillateur pour une élévation proportionelle de la fréquence de 1.9 MHz à 3 à 4 MHz, au moyen de l'électrode 45 reliée à la self L3 - L4, ce qui a pour effet d'inclure l'impédance du corps d'un patient à celle des bobines de la self L3 - L4.

Par rapport aux circuits conventionnels utilisés dans ce domaine, pilotés par quartz, et qui fonctionnent à fréquence fixe, ce circuit fournit un courant haute fréquence émetteur pur, donc exempt d'harmoniques, ce qui assure une transmission efficace par les points 15 de la sonde 1. Toutefois, il est également avantageux d'employer ce circuit pour d'autres applications non couvertes par les revendications jointes, notamment l'épilation haute fréquence au moyen d'une aiguille. Donc, son champ d'application n'est pas limité au procédé employant un gel conducteur chargé.

La commande de fonctionnement du circuit oscillateur 40 est faite, soit par l'actionnement d'une pédale 38, soit par commande manuelle du circuit temporisé d'arrêt automatique 50 et la commutation du circuit optoélectronique 52. Dans le premier cas, la sonde 1 émet, tant que l'on appuie sur la pédale 38. Dans le second cas, utilisé, par exemple, lorsque la sonde 1 est maintenue immobile sur un endroit de la peau, la sonde émet pendant une durée pré-réglée. En option, on peut prévoir un compteur horaire pour déterminer la durée du travail.

L'action synergétique obtenue avec le mélange de gel et de produit traitant selon l'invention ressort clairement des exemples ci-dessous.

### Exemple I (Comparatif)

Un gel conducteur ELECTROGEL™ fabriqué par la société Diaplix et utilisé pour l'échographie et l'électrocardiographie a été appliqué sur une zone pileuse de la peau mesurant environ 5cm x 5cm. Au moyen de l'appareil décrit selon l'invention et tel qu'illustré dans les dessins, un courant électromagnétique à une fréquence de 2MHz a été appliqué pendant une durée de 10 à 20 minutes. Aucun effet n'a été constaté. Le gel n'a pas pénétré dans les pores ni produit une modification de résistance du poil à l'extraction.

### Exemple II (Comparatif)

Une lotion post-épilatoire BANNIPIL™ a été appliquée sur une zone pileuse de la peau mesurant environ 5cm x 5cm, et soumise au même traitement par l'appareil décrit selon l'invention. Ce produit post-épilatoire est à base d'extraits de plantes (bouleau, prêle et hamamélis) et d'huiles essentielles (produit de distillation de citron, verveine et lavande) et comprend en outre du propylène glycol, des acides gras, des polyoxyéthylènes et de l'eau déminéralisée. A la fin du traitement, aucun effet n'a été constaté. Le produit n'a pas pénétré dans les pores. La racine du poil n'a pas été atteinte. La résistance du poil à l'extraction n'a pas été modifiée.

### Exemple III

Un mélange de 89 vol% de gel ELECTROGEL™, 9 vol% d'une lotion post-épilatoire BANNIPIL™, et 2 vol% d'alcool médicinal à 90° contenant du NaCl, a été préparé. Ce mélange a été appliqué sur une zone pileuse de la peau mesurant 5cm x 5cm. Au moyen de l'appareil décrit selon l'invention, un courant électromagnétique à une fréquence de 2MHz a été appliqué pendant une durée de 10 à 20 minutes comme auparavant. On a constaté une pénétration de la lotion dans les pores. Cette pénétration a permis une extraction du poil sans résistance. Un examen de la racine au microscope (agrandissement 150 fois) fait apparaître que la structure de la racine du poil est lisse, ramollie et rétrécie, ce qui indique que la lotion a pénétré dans le pore et qu'elle a eu un effet. Cet effet est d'enlever la membrane kératinisante du poil, ce qui facilite son extraction.

### Exemple IV

Un mélange de 47.5 vol% de gel ELECTROGEL™ et 47.5 vol% de minoxydil et 5 vol% d'alcool médicinal à 90° contenant du NaCl a été préparé. Ce mélange a été appliqué sur une zone pileuse de la peau d'une pilosité de départ de 5 à 10% de la pilosité normale du cuir chevelu, mesurant environ 5cm x 10cm. Au moyen de l'appareil décrit selon l'invention, un courant électromagnétique à une fréquence de 2MHz a été appliqué pendant une durée de 15 à 20 minutes. Ce traitement a été effectué pendant 3 semaines à raison de deux applications par semaine, puis après avoir observé un repos de 15 jours, on a repris le traitement à raison d'une application par semaine pendant 8 semaines. A la fin du traitement, la pilosité était de 15-20% de la pilosité normale du cuir chevelu, et la qualité du poil était supérieure au niveau de la résistance mécanique et de la dureté du poil. La couleur du poil était également plus foncée. Ce traitement a donc eu un effet nutritif et a amélioré la vitalité du poil. Ce résultat représente une amélioration substantielle par rapport à ce que l'on peut obtenir par la seule application, sur la surface du cuir chevelu, de minoxydil ou d'un autre produit favorisant la pousse des cheveux, avec ou sans application d'un courant HF.

## Revendications

1. Procédé de traitement cosmétique par l'application sur la peau d'un produit traitant destiné à agir sur les racines des poils ou des cheveux, et l'application d'un courant électromagnétique de haute fréquence, caractérisé en ce que l'on applique sur la peau (2) un mélange de ce produit traitant et de gel conducteur non-polymérisable (3) d'un type utilisé usuellement pour le couplage ultrasonique de sondes avec la peau, et que l'on émet dans ce mélange le courant électromagnétique de haute fréquence pour amener le produit contenu dans une solution conductrice provenant du mélange à pénétrer dans les pores de la peau jusqu'aux racines des poils ou des cheveux.

2. Procédé selon la revendication 1 pour une épilation longue durée, caractérisé en ce que l'on applique sur la peau (2) un mélange dudit gel conducteur (3) et d'un produit à effet d'atrophie sur les racines des poils, que l'on fait pénétrer ce produit dans les pores de la peau jusqu'aux racines des poils par l'émission haute fréquence, et que l'on enlève ensuite les poils par des moyens idoines.

3. Procédé selon la revendication 1 pour un traitement cosmétique de la calvitie, caractérisé en ce que l'on applique sur le cuir chevelu un mélange dudit gel conducteur (3) et d'un produit régénérateur des cheveux, et que l'on fait pénétrer ce produit dans les pores du cuir chevelu jusqu'aux racines des cheveux par l'émission haute fréquence.

4. Procédé selon l'une quelconque des revendications 1, 2 et 3, caractérisé en ce que l'on émet dans le mélange (3), par une ou plusieurs rangées de points d'émission électromagnétiques distincts (15), un courant haute fréquence émetteur pur.

5. Appareil de mise en oeuvre du procédé selon l'une quelconque des revendications 1 à 4 pour le traitement de la peau (2) par induction transcutanée d'un courant électromagnétique de haute fréquence à l'aide d'un gel conducteur non-polymérisable (3) d'un type utilisé usuellement pour le couplage ultrasonique de sondes avec la peau, chargé de produit traitant et appliqué sur la peau à l'endroit voulu, le courant électromagnétique amenant ce produit à pénétrer les pores de la peau, caractérisé en ce que ledit appareil comprend:
- un organe maniable de contact (1) avec la peau (2) ayant un corps non-conducteur (10,12) pourvu d'une surface d'appui (17), destinée à être appliquée sur la peau, cette surface (17) comportant une pluralité de points conducteurs d'émission électromagnétique distincts (15), ces points (15) débouchant par des orifices dans cette surface, de préférence en retrait par rapport à celle-ci, de manière que, en cours d'utilisation de l'appareil, ils puissent contacter du gel conducteur chargé (3) appliqué sur la peau,
- et un circuit électrique (27) apte à fournir aux points d'émission électromagnétique (15) du courant à haute fréquence qui, en cours d'utilisation de l'appareil, est émis par lesdits points à travers le gel conducteur chargé (3).

6. Appareil selon la revendication 5, caractérisé en ce que l'organe de contact (1) a une surface d'appui oblongue (17) dans laquelle lesdits points (15) sont disposés en retrait, alignés en une ou plusieurs rangées.

7. Appareil selon l'une quelconque des revendications 5 et 6, caractérisé en ce que lesdits points (15) sont formés par des parties exposées de spires d'un solénoïde (12) noyé dans le corps (10) de l'organe de contact.

8. Appareil selon l'une quelconque des revendications 5 à 7, caractérisé en ce que le circuit (27) comprend un circuit oscillant haute fréquence de puissance (40), comportant un transistor (TR1) monté en oscillateur de puissance en combinaison avec des bobines de self inductance double spirale en carré (L1 - L2; L3 - L4).

9. Appareil selon la revendication 8, caractérisé en ce qu'il comporte une électrode (45) permettant d'ajouter l'impédance du corps d'une personne traitée à celle des bobines de self inductance (L3 - L4) de manière à éléver la fréquence du courant émetteur lors de l'utilisation.

10. Appareil selon l'une quelconque des revendications 8 et 9, caractérisé en ce que le circuit (27) comporte un potentiomètre (43) pour le réglage de l'intensité du courant émetteur.

11. Appareil selon l'une quelconque des revendications 5 à 10, caractérisé en ce qu'il comporte plusieurs organes de contact (1) interchangeables, ayant des surfaces d'appui de forme et/ou de grandeur différente.

12. Appareil selon l'une quelconque des revendications 5 à 11, caractérisé en ce qu'il comporte en outre un embout à aiguille interchangeable avec ledit ou lesdits organe(s) de contact (1).

13. Utilisation de l'appareil selon l'une quelconque des revendications 5 à 11, pour l'épilation cosmétique de longue durée, caractérisée en ce que l'on applique ladite surface d'appui (17) sur la peau à traiter (2), préalablement recouverte de gel conducteur non-polymérisable (3) d'un type utilisé usuellement pour le couplage ultrasonique de sondes avec la peau, chargé d'un produit à effet d'atrophie sur les racines des poils, pour amener le produit à pénétrer dans les pores de la peau jusqu'aux racines des poils par l'émission haute fréquence émise par lesdits points (15), les poils étant ensuite enlevés par des moyens idoines.

14. Utilisation de l'appareil selon l'une quelconque des revendications 5 à 11, pour le traitement cosmétique de la calvitie, caractérisée en ce que l'on applique ladite surface d'appui (17) sur le cuir chevelu à traiter (2), préalablement recouverte de gel conducteur non-polymérisable (3) d'un type utilisé usuellement pour le couplage ultrasonique de sondes avec la peau, chargé d'un produit régénérateur des cheveux, pour amener le produit à pénétrer dans les pores du cuir chevelu jusqu'aux racines des cheveux par l'émission haute fréquence émise par lesdits points (15).

15. Utilisation cosmétique d'un mélange d'un gel conducteur non-polymérisable d'un type utilisé usuellement pour le couplage ultrasonique de sondes avec la peau, et d'un produit de traitement cosmétique bio-actif, comme interface conducteur entre une source d'émission de courant électromagnétique de haute fréquence et la peau et comme moyen de transport du produit bio-actif pour amener celui-ci, contenu dans une solution conductrice provenant du mélange, à pénétrer dans les pores de la peau jusqu'aux racines des poils ou des cheveux.

16. Utilisation selon la revendication 15 pour l'épilation longue durée, d'un mélange du gel conducteur et d'une lotion à effet d'atrophie sur les racines des poils comportant des extraits de plantes et des huiles essentielles.

17. Utilisation selon la revendication 15 pour un traitement de repousse de cheveux, d'un mélange du gel conducteur et d'un produit favorisant la pousse des cheveux.

18. Utilisation selon l'une quelconque des revendications 15 à 17, d'un mélange comprenant en outre un agent pour améliorer la conductivité électrique du produit, en particulier de l'alcool et/ou du chlorure de sodium.

19. Mélange d'un gel conducteur et d'un produit de traitement cosmétique bio-actif, utilisable dans un procédé d'épilation longue durée selon la revendication 2 ou avec l'appareil selon l'une quelconque des revendications 5 à 12 ou utilisable selon la revendication 13 comme moyen de transport du produit bio-actif pour amener celui-ci, contenu dans une solution conductrice provenant du mélange, a pénétrer dans les pores de la peau jusqu'aux racines des poils lorsque le mélange est appliqué sur la peau, ce mélange comportant un gel conducteur non-polymérisable d'un type utilisé usuellement pour le couplage ultrasonique de sondes avec la peau, par exemple à base d'un polymère de l'ester 5-bromo-5-nitro-1,3-dioxane-2-carboxyvinylique, et une lotion à effet d'atrophie sur les racines des poils comportant des extraits de plantes et des huiles essentielles.

20. Mélange d'un gel conducteur et d'un produit de traitement cosmétique bio-actif, utilisable dans un procédé de traitement de la calvitie selon la revendication 3 ou avec l'appareil selon l'une quelconque des revendications 5 à 12 ou utilisable selon la revendication 14 comme moyen de transport du produit bio-actif pour amener celui-ci, contenu dans une solution conductrice provenant du mélange, à pénétrer dans les pores de la peau jusqu'aux racines des cheveux lorsque le mélange est appliqué sur le cuir chevelu, ce mélange comportant un gel conducteur non-polymérisable d'un type utilisé usuellement pour le couplage ultrasonique de sondes avec la peau, par exemple à base d'un polymère de l'ester 5-bromo-5-nitro-1,3-dioxane-2-carboxyvinylique, et un produit favorisant la pousse des cheveux.

21. Mélange selon la revendication 19 ou 20, comprenant en outre un agent pour améliorer la conductivité électrique du produit, en particulier de l'alcool et/ou du chlorure de sodium.

## Claims

1. A method of cosmetic treatment by applying to the skin a treating product able to act on the hair roots and by applying a high-frequency electromagnetic current, characterised in that a mixture of this treating product and of a non polymerisable conductive gel (3) of a type used usually for coupling ultrasound probes with the skin is applied to the skin (2), and the high-frequency electromagnetic current is emitted into this mixture to cause the product, within a conductive solution derived from the mixture, to penetrate into the pores of the skin down to the hair roots.

2. A method according to claim 1 for long lasting depilation, characterised in that a mixture of said conductive gel (3) and of a product producing atrophy of the hair roots is applied to the skin (2), this product is caused to penetrate into the pores of the skin down to the hair roots by the high-frequency emission, and the hairs are then removed by suitable means.

3. A method according to claim 1 for cosmetic treatment of baldness, characterised in that a mixture of said conductive gel (3) and of a hair regenerating product is applied to the scalp, and this product is caused by the high-frequency emission to penetrate into the pores of the scalp down to the hair roots.

4. A method according to any one of claims 1, 2 and 3, characterised in that a pure high-frequency emissive current is emitted into the mixture (3) by one or several rows of discrete electromagnetic emission points (15).

5. An apparatus for carrying out the method according to any one of claims 1 to 4 for treatment of the skin (2) by transcutaneous induction of a high-frequency electromagnetic current by means of a non-polymerisable conductive gel (3) of a type used usually for coupling ultrasound probes with the skin, loaded with treating product and applied to the skin at the desired location, the electromagnetic current causing this product to penetrate into the pores of the skin, characterised in that said apparatus comprises :
- a handleable member (1) for contacting the skin (2), having a non-conductive body (10,13) provided with a contact surface (17) adapted to be applied to the skin, this surface (17) having a plurality of discrete conductive electromagnetic emission points (15), these points (15) being exposed through openings in this surface, and which are preferably recessed with respect to the latter, so that during use of the apparatus these points may contact loaded conductive gel (3) applied to the skin,
- and an electric circuit (27) able to supply to the electromagnetic emission points (15) high-frequency current which, during use of the apparatus, is emitted by said points through the loaded conductive gel (3).

6. An apparatus according to claim 5, characterised in that the contact member (1) has an oblong contact surface (17) wherein said points (15), which are aligned in one or several rows, are recessed.

7. An apparatus according to either of claims 5 and 6, characterised in that said points (15) are formed by exposed parts of turns of a solenoid (12) embedded in the body (10) of the contact member.

8. An apparatus according to any one of claims 5 to 7, characterised in that the circuit (27) comprises a high-frequency oscillatory power-circuit (40) including a transistor (TR1) connected as a power oscillator in combination with double-square-winding self-inductance coils (L1 - L2; L3 - L4).

9. An apparatus according to claim 8, characterised in that it comprises an electrode (45) by means of which the impedance of a treated person's body may be added to that of the self-inductance coils (L3 - L4) to increase the frequency of the emissive current during use.

10. An apparatus according to either of claims 8 and 9, characterised in that the circuit (27) includes a potentiometer (43) for adjusting the value of the emissive current.

11. An apparatus according to any one of claims 5 to 10, characterised in that it includes several interchangeable handleable members (1) having contact surfaces of different shapes and/or different sizes.

12. An apparatus according to any one of claims 5 to 11, characterised in that it further includes a needle fitment interchangeable with said handleable member or members (1).

13. Use of the apparatus according to any one of claims 5 to 11, for long lasting cosmetic depilation, characterised in that said contact surface (17) is applied to the skin to be treated (2), previously covered with non-polymerisable conductive gel (3) of a type used usually for the ultrasonic coupling of probes with the skin, loaded with a product able to atrophy the hair roots, to cause the product to penetrate into the pores of the skin down to the hair roots by the high-frequency emissive current emitted by said points (15), the hair being removed afterwards by suitable means.

14. Use of the apparatus according to any one of claims 5 to 11, for the cosmetic treatment of baldness, characterised in that said contact surface (17) is applied to the scalp to be treated (2), previously covered with non-polymerisable conductive gel (3) of a type used usually for the ultrasonic coupling of probes with the skin, loaded with a hair-regenerating product, to cause the product to penetrate into the pores of the scalp down to the hair-roots by the high-frequency emissive current emitted by said points (15).

15. Cosmetic use of a mixture of a non-polymerisable conductive gel (3) of a type used usually for coupling ultrasound probes with the skin, and of a cosmetic bioactive treatment product, as a conductive interface between an emitting source of a high-frequency electromagnetic current and the skin, and as a means for transporting the bioactive product to cause the latter, within a conductive solution derived from the mixture, to penetrate into the pores of the skin down to the hair roots.

16. Use according to claim 15, for long lasting depilation, of a mixture of the conductive gel and of a lotion able to atrophy the hair-roots, comprising plant extracts and essential oils.

17. Use according to claim 15, for a hair-regrowth treatment, of a mixture of the conductive gel and a hair-growth product.

18. Use according to any one of claims 15 to 17, of a mixture further comprising an agent to improve the electrical conductivity of the product, in particular alcohol and/or sodium chloride.

19. Mixture of a conductive gel and of a bioactive cosmetic treatment product, usable in a long lasting depilation method according to claim 2, or with the apparatus according to any one of claims 5 to 12, or usable according to claim 13 as a means for transporting the bioactive product to cause the latter, within a conductive solution derived from the mixture, to penetrate into the pores of the skin down to the hair roots when the mixture is applied to the skin, this mixture comprising a non-polymerisable conductive gel of a type used usually for coupling ultrasound probes with the skin, for example based on a polymer of 5-bromo-5-nitro-1,3-dioxane-2-carboxyvinilic ester, and a lotion able to atrophy the hair roots comprising plant extracts and essential oils.

20. Mixture of a conductive gel and of a bioactive cosmetic treatment product, usable in a baldness treatment method according to claim 3, or with the apparatus according to any one of claims 5 to 12, or usable according to claim 14 as a means for transporting the bioactive product to cause the latter, within a conductive solution derived from the mixture, to penetrate into the pores of the skin, down to the hair roots when the mixture is applied to the scalp, this mixture comprising a non-polymerisable conductive gel of a type used usually for coupling ultrasound probes with the skin, for example based on a polymer of 5-bromo-5-nitro-1,3-dioxane-2-carboxyvinilic ester, and a hair growth product.

21. Mixture according to claim 19 or 20, further comprising an agent to improve the electrical conductivity of the product, in particular alcohol and/or sodium chloride.

## Patentansprüche

1. Verfahren für eine kosmetische Behandlung durch Auftragen eines Behandlungsprodukts auf die Haut, welches imstande ist, auf die Haarwurzeln einzuwirken und durch Anlegen eines hochfrequenten, elektromagnetischen Stroms, dadurch gekennzeichnet, dass eine Mixtur dieses Behandlungsprodukts und eines nichtpolymerisierbaren, leitenden Gels (3) jener Art, die üblicherweise zur Ultraschallkopplung von Sonden mit der Haut verwendet wird, auf die Haut (2) aufgetragen wird, und dass der hochfrequente, elektromagnetische Strom in die Mixtur ausgesandt wird, wodurch das Produkt innerhalb einer aus der Mixtur gewonnenen, leitenden Lösung in die Hautporen bis zu den Hautwurzeln eindringt.

2. Verfahren nach Anspruch 1 zu einer fortdauernden Enthaarung, dadurch gekennzeichnet, dass eine Mixtur des genannten leitenden Gels (3) und eines eine Haarwurzel-Atrophie verursachenden Produkts auf die Haut (2) aufgetragen wird,und dass dieses Produkt durch die hochfrequente Emission veranlasst wird, in die Hautporen bis zu den Haarwurzeln einzudringen, wonach die Haare durch ein geeignetes Mittel entfernt werden.

3. Verfahren nach Anspruch 1 für eine kosmetische Behandlung von Kahlheit, dadurch gekennzeichnet, dass eine Mixtur des genannten leitenden Gels (3) und eines haarregenerierenden Produkts auf die Kopfhaut aufgetragen wird, und dass dieses Produkt durch die hochfrequente Emission veranlasst wird, in die Hautporen bis zu den Haarwurzeln einzudringen.

4. Verfahren nach irgendeinem der Ansprüche 1, 2 und 3, dadurch gekennzeichnet, dass ein reiner Hochfrequenz-Emissionsstrom in die Mixtur aus einer oder einer Mehrzahl von Reihen diskreter, elektromagnetischer Emissionspunkte (15) ausgesandt wird.

5. Vorrichtung zur Durchführung des Verfahrens gemäss irgendeinem der Ansprüche 1 bis 4 zur Behandlung der Haut (2) durch transkutane Induktion eines hochfrequenten, elektromagnetischen Stroms mittels eines nichtpolymerisierbaren, leitenden Gels (3) jener Art, die üblicherweise zur Ultraschallkopplung von Sonden mit der Haut verwendet wird, geladen mit einem Behandlungsprodukt, wobei dieses auf die gewünschte Hautstelle aufgetragen wird, und wobei der elektromagnetische Strom das Produkt in die Hautporen befördert, dadurch gekennzeichnet, dass die Vorrichtung enthält:
- ein handliches Glied (1) zur Kontaktaufnahme mit der Haut (2), enthaltend einen nichtleitenden, mit einer Kontaktfläche (17) versehenen Körper (10, 12) zum Anlegen auf die Haut, wobei die Kontaktfläche (17) eine Mehrzahl diskreter, leitender, elektromagnetischer Emissionspunkte (15) aufweist, die durch Öffnungen in der erwähnten Fläche frei- und vorzugsweise in Bezug auf die Fläche vertieft liegen, so dass diese Punkte während des Einsatzes der Vorrichtung das auf die Haut aufgetragene, geladene, leitende Gel (3) kontaktieren können,
- und einen elektrischen Stromkreis (27) zur Versorgung der elektromagnetischen Emissionspunkte (15) mit hochfrequentem Strom, der während des Einsatzes der Vorrichtung von diesen Punkten aus das geladene, leitende Gel (3) durchströmt.

6. Vorrichtung nach Anspruch 5, dadurch gekennzeichnet, dass das handliche Glied (1) eine längliche Kontaktfläche (17) aufweist, in welcher die erwähnten, in mindestens einer Reihe ausgerichteten Punkte (15) vertieft angeordnet sind.

7. Vorrichtung nach irgendeinem der Ansprüche 5 und 6, dadurch gekennzeichnet, dass die erwähnten Punkte (15) durch freiliegende Teile von Windungen eines im Körper (10) des handlichen Gliedes eingebetteten Solenoids (12) gebildet sind.

8. Vorrichtung nach irgendeinem der Ansprüche 5 bis 7, dadurch gekennzeichnet, dass der Stromkreis (27) eine hochfequent oszillierende Leistungsschaltung (40) aufweist, enthaltend einen als Leistungsoszillator in Kombination mit Selbstinduktionsspulen (L1 - L2; L3 - L4), je bestehend aus einem Paar rechteckförmig gewickelter Spulen, geschalteten Transistor (TR1).

9. Vorrichtung nach Anspruch 8, dadurch gekennzeichnet, dass eine Elektrode (45), durch welche die Körperimpedanz der behandelten Person zu jener der Spulen (L3 - L4) hinzuzählbar ist, um die Frequenz des Emissionsstroms während der Benutzung zu erhöhen, vorgesehen ist.

10. Vorrichtung nach irgendeinem der Ansprüche 8 und 9, dadurch gekennzeichnet, dass der Stromkreis (27) ein Potentiometer (43) zur Einstellung des Emissionsstrom-Wertes enthält.

11. Vorrichtung nach irgendeinem der Ansprüche 5 bis 10, dadurch gekennzeichnet, dass sie eine Mehrzahl auswechselbarer handlicher Glieder (1) enthält, deren Kontaktflächen verschiedene Formen und/oder verschiedene Grössen aufweisen.

12. Vorrichtung nach irgendeinem der Ansprüche 5 bis 11, dadurch gekennzeichnet, dass weiter eine nadelförmige Anordnung vorgesehen ist, die mit dem handlichen Glied oder den handlichen Gliedern (1) austauschbar ist.

13. Verwendung der Vorrichtung nach irgendeinem der Ansprüche 5 bis 11 für eine fortdauernde, kosmetische Enthaarung, dadurch gekennzeichnet, dass die Kontaktfläche (17) an die zu behandelnde Haut (2) angelegt wird, auf welche vorher ein nichtpolymerisierbares Gel (3) jener Art, wie sie üblicherweise zur Ultraschallkopplung von Sonden mit der Haut verwendet wird, geladen mit einem eine Haarwurzel-Atrophie verursachenden Produkt, aufgetragen wurde, um das Produkt in die Hautporen bis zu den Haarwurzeln mittels eines hochfrequenten Emissionsstroms, der aus den erwähnten Punkten (15) ausgesandt wurde, eindringen zu lassen, wobei die Haare nachfolgend durch geeignete Mittel entfernt werden.

14. Verwendung der Vorrichtung nach irgendeinem der Ansprüche 5 bis 11 für eine kosmetische Behandlung von Kahlheit, dadurch gekennzeichnet, dass die Kontaktfläche (17) an die zu behandelnde Kopfhaut (2) angelegt wird, auf welche vorher ein nichtpolymerisierbares Gel (3) jener Art, wie sie üblicherweise zur Ultraschallkopplung von Sonden mit der Haut verwendet wird, geladen mit einem haarregenerierenden Produkt, aufgetragen wurde, um das Produkt in die Haarporen bis zu den Haarwurzeln mittels eines hochfrequenten Emissionsstroms, der aus den erwähnten Punkten (15) ausgesandt wurde, eindringen zu lassen.

15. Kosmetische Verwendung einer Mixtur aus einem nichtpolymerisierbaren, leitenden Gel (3) jener Art, wie sie üblicherweise zur Ultraschallkopplung von Sonden mit der Haut verwendet wird, und einem kosmetischen, bioaktiven Behandlungsprodukt, als eine leitende Zwischenschicht zwischen einer, hochfrequenten elektromagnetischen Strom emittierenden Quelle und der Haut, und als Mittel zur Beförderung des bioaktiven Produkts, um dieses innerhalb einer, aus der Mixtur gewonnenen, leitenden Lösung in die Hautporen bis zu den Haarwurzeln eindringen zu lassen.

16. Verwendung nach Anspruch 15 einer Mixtur aus dem leitenden Gel und einer Haarwurzel-Atrophie verursachenden Lotion zur fortdauernden Enthaarung, enthaltend Pflanzenextrakte und ätherische Öle.

17. Verwendung nach Anspruch 15 einer Mixtur aus dem leitenden Gel und einem den Haarwuchs fördernden Produkt zur haarregenerierenden Behandlung.

18. Verwendung nach irgendeinem der Ansprüche 15 bis 17 einer Mixtur, die weiter ein Agens zur Verbesserung der elektrischen Leitfähigkeit des Produkts enthält, insbesondere Alkohol und/oder Natriumchlorid.

19. Mixtur aus einem leitenden Gel und einem bioaktiven Behandlungsprodukt, verwendbar in einem Verfahren zur fortdauernden Enthaarung gemäss Anspruch 2, oder mit der Vorrichtung nach irgendeinem der Ansprüche 5 bis 12, oder verwendbar nach Anspruch 13 als Mittel zur Beförderung des bioaktiven Produkts, um es Innerhalb der, aus der Mixtur gewonnenen, leitenden Lösung in die Hautporen bis zu den Haarwurzeln eindringen zu lassen, wenn die Mixtur auf die Haut aufgetragen wird, wobei diese Mixtur ein nichtpolymerisierbares, leitendes Gel beinhaltet von jener Art, wie sie üblicherweise zur Ultraschallkopplung von Sonden mit der Haut verwendet wird, zum Beispiel basierend auf einem Polymer eines 5-bromo-5-nitro-1,3-dioxan-2-carboxyvinilesters, und eine Haarwurzeln atrophierende Lotion, enthaltend Pflanzextrakte und ätherische Öle.

20. Mixtur aus einem leitenden Gel und einem bioaktiven, kosmetischen Behandlungsprodukt, verwendbar in einem Kahlheitsbehandlungsverfahren nach Anspruch 3, oder mit der Vorrichtung nach irgendeinem der Ansprüche 5 bis 12, oder verwendbar nach Anspruch 14 als Mittel zur Beförderung des bioaktiven Produkts, um dieses innerhalb einer leitenden, aus der Mixtur gewonnenen Lösung in die Hautporen bis zu den Hautwurzeln eindringen zu lassen, wenn die Mixtur auf die Kopfhaut aufgetragen wird, wobei diese Mixtur ein nichtpolymerisierbares , leitendes Gel beinhaltet von jener Art, wie sie üblicherweise zur Ultraschallkopplung von Sonden mit der Haut verwendet wird, zum Beispiel basierend auf einem Polymer eines 5-bromo-5-nitro-1,3-dioxan-2-carboxyvinilesters, und ein den Haarwuchs förderndes Produkt.

21. Mixtur nach Anspruch 19 oder 20, die weiter ein Agens zur Verbesserung der elektrischen Leitfähigkeit des Produkts enthält, insbesondere Alkohol und/oder Natriumchlorid.
